# DEMANDE DE BREVET EUROPEEN

(11) **EP 0 841 046 A1**
(43) Date de publication de la demande: **13.05.1998**
(21) Numéro de dépôt: 97119040.0
(22) Date de dépôt: 31.10.1997
(51) Int. Cl.: A61F 7/10

(54) **Dispositif d'abaissement local de la temperature du corps et notamment des membres**

(30) Priorité: 08.11.1996 FR 9613843; 17.10.1997 FR 9712993
(71) Demandeur: Blanc, Catherine, 81000 Albi (FR); Rossignol, Pierre, 81000 Albi (FR)
(72) Inventeur: Blanc, Catherine, 81000 Albi (FR); Rossignol, Pierre, 81000 Albi (FR)
(74) Mandataire: Schlawick, Yvan

(57) **Abrégé**

L'invention concerne un dispositif I d'abaissement local de la température du corps du type de celui, constitué par deux parois 100 et 200 qui, adoptant la forme d'un secteur angulaire d'une couronne en position déployée et une forme tronconique en position enroulée, sont liées entre elles de façon à former au moins un compartiment 300 de stockage pour une matière à inertie thermique, lesdites parois 100 et 200 étant liées entre elles de façon à former au moyen de leurs zones de liaison un ou plusieurs compartiments 300 étanches ou non entre eux, remarquable en ce qu'il présente au niveau de sa partie centrale en position enroulée une forme convexe sensiblement identique au profil convexe d'un molletlui permettant d'épouser ledit profil et ainsi de soigner par le froid dans les meilleures conditions.

Applications : traitement des insuffisances veineuses, des thrombophlébites superficielles et des oedèmes traumatiques.

## Description

La présente invention a trait au domaine médical et concerne plus particulièrement les adaptations permettant d'abaisser superficiellement et localement la température de la jambe, dans le cadre d'un traitement des insuffisances veineuses et des thrombophlébites superficielles.

En effet, un des traitements appropriés pour atténuer les douleurs dues à une insuffisance veineuse ou une thrombophlébite superficielle consiste à abaisser localement la partie du corps atteinte (généralement les jambes).

Ces dernières années ont vu apparaître quelques dispositifs pour applications thérapeutiques de ce type :

Ainsi, la compresse décrite dans le brevet européen n° 0 567 724 AP consiste en une pluralité de poches en lin ou en coton liées entre elles par des bandes à fermeture "Velcro" et disposées entre elles de façon à laisser un espace ou une couture pour donner une structure souple à l'ensemble de la compresse. Des sachets souples contenant une matière de conservation du froid à grande inertie thermique (par matière de conservation du froid nous entendons tout type de matière pouvant être refroidie par congélation et qui reste froide au toucher pendant un temps suffisamment long et dont la température est de -20°c) sont introduits dans les poches par une ouverture équipée d'une fermeture "éclair". La compresse, destinée à être plaquée sur la zone du corps thrombosée, a pour inconvénient non négligeable de ne permettre qu'un enveloppement cylindrique des zones à traiter et n'a donc pas une forme très adaptée à une application homogène sur un bras, une cuisse ou un mollet.

Un autre dispositif décrit dans le brevet français n° 2.143.918 est constitué par une poche en matière plastique retenant un gel hydrophile restant froid longtemps. Cette poche, se présentant sous la forme d'une bande rectangulaire est reliée à ses deux extrémités par une bande élastique assurant le plaquage de la poche sur la zone du corps à traiter. En outre, les deux parois de ce dispositif de refroidissement sont d'épaisseur et de conductivité thermique différentes afin de proposer deux températures selon le sens d'application sur le corps. Le gel et donc la poche restent souples même lorsque le gel est congelé permettant ainsi à la poche de prendre n'importe quelle forme géométrique. Ce dispositif comme la compresse du brevet européen précédent, a pour désavantage de ne pouvoir proposer qu'une forme d'enveloppement cylindrique et se trouve donc mal adapté au fuselage naturel des membres du corps humain.

Un autre dispositif décrit dans le brevet français n° 2.063.258, propose quant a lui, une ceinture réfrigérante comportant plusieurs compartiments indépendants reliés entre eux mais présentant toujours une forme déployée rectangulaire qui ne lui permette d'avoir qu'une forme d'enveloppement cylindrique toujours mal adaptée aux contours fuselés des zones thrombosées des jambes, bras, cuisses du corps humain.

Pour résoudre cet enveloppement inadéquat de la zone thrombosée, plusieurs dispositifs de l'art antérieur utilisent la forme d'un secteur angulaire d'une couronne en position déployée et donc une forme tronconique en position enroulée permettant de s'adapter au fuselage naturel des membres du corps et notamment des jambes. Bien qu'apportant un plus indéniable, la forme tronconique est difficilement adaptable au profil convexe du mollet obligeant l'utilisateur à serrer au maximum ledit dispositif pour éviter sa désolidarisation de la jambe, ce qui peut avoir un effet contraire aux soins demandés. De plus la forme tronconique seule n'est pas esthétique et est décelable même sous un vêtement.

Partant de cet état de fait, les demandeurs ont donc mené des recherches visant à améliorer le principe de compresse pour application du froid en particulier celui des compresses destinées au traitement des membres du corps humain. Ces recherches ont abouti à la conception d'une forme originale de dispositif d'abaissement local de la température du corps et notamment des membres permettant d'obvier aux inconvénients précités des dispositifs de refroidissement tout en s'adaptant dans les meilleures conditions possibles à la forme fuselée des membres du corps humain et notamment à la jambe.

Selon les dispositions principales de l'invention, ce dispositif d'abaissement local de la température du corps du type de celui, constitué par deux parois qui, adoptant la forme d'un secteur angulaire d'une couronne en position déployée et une forme tronconique en position enroulée, sont liées entre elles de façon à former au moins un compartiment de stockage pour une matière à inertie thermique, lesdites parois étant liées entre elles de façon à former au moyen de leurs zones de liaison un ou plusieurs compartiments étanches ou non entre eux.

Ce dispositif est remarquable en ce qu'il présente au niveau de sa partie centrale en position enroulée une forme convexe sensiblement identique au profil convexe d'un mollet.

Cette forme préférentielle adoptée par le dispositif de l'invention a l'avantage de former, fermée et enroulée autour de la partie à traiter, un fuseau, lorsque ses bords extrêmes sont réunis pour s'adapter au plus près du fuselage naturel des membres inférieurs ou supérieurs. Ceci est d'autant plus important que les thrombophlébites superficielles apparaissent très souvent dans les membres inférieurs du patient. Ainsi, le galbe créé par la forme convexe permet de s'adapter parfaitement à la forme du mollet offrant ainsi des avantages que les dispositifs de l'art antérieur ne pouvaient offrir, et parmi ceux-ci :
- un dispositif d'abaissement de la température moulant, permettant non seulement de mieux répartir le froid et donc de mieux soigner mais aussi d'être beaucoup plus esthétique,
- un dispositif ne se voyant pas sous un vêtement et permettant donc à l'utilisateur de marcher sans entrave contrairement aux autres dispositifs qui, même tronconiques une fois enroulés, n'autorisaient pas une marche naturelle.

Selon une caractéristique particulièrement avantageuse de l'invention,la matière à grande inertie thermique se présente sous la forme d'un gel de polyuréthane. Particulièrement adapté pour une telle application, le gel de polyuréthane autorise à partir d'une petite épaisseur, une diffusion du froid plus longue du fait de ses caractéristiques thermiques qui lui permettent après réfrigération de garder sa température très longtemps pour soigner par le froid dans les meilleures conditions.

Bien entendu cette matière à grande inertie thermique n'est pas appliquée directement sur la peau, car cette dernière est, selon une autre caractéristique particulièrement avantageuse de l'invention, contenue dans une poche hermétique en thermoplastique extensible du type polyéthyléne réalisée par thermo-soudure. Le plastique est le meilleur moyen de créer un compartiment fin et étanche par thermo-soudure pour éviter toute sur-épaisseur et diffusant ainsi directement et le plus efficacement possible sa température au corps avec lequel il est en contact. Un autre avantage de l'utilisation du plastique et ici du polyéthylène extensible est que la poche hermétique s'adapte à n'importe quel profil ou volume assurant un "moulage" de la zone à traiter.

Selon une autre caractéristique de l'invention, la poche hermétique thermo-soudée contenant ladite matière à inertie thermique est placée à l'intérieur du dispositif dans le compartiment constitué par les deux parois formant housse et adoptant le profil convexe du dispositif. Pour des raisons de confort et de faisabilité, l'opération qui consiste à placer une poche hermétique en plastique adoptant toutes les formes contenant la matière à grande inertie thermique à l'intérieur d'une housse adoptant un profit convexe et sensiblement tronconique en position enroulée, est une solution particulièrement avantageuse. En effet, le contact sur la peau d'un autre matériau que le plastique est plus agréable. De plus, il est plus facile de tailler dans, par exemple, du tissu un secteur angulaire de couronne et lier les éléments entre eux puis introduire une poche hermétique rectangulaire thermo-soudée que de réaliser un secteur angulaire de couronne dans du plastique et assurer la thermo-soudure sur des lignes en courbes.

Les concepts fondamentaux de l'invention venant d'être exposés ci-dessus dans leur forme la plus élémentaire, d'autres détails et d'autres caractéristiques de la présente invention ressortiront plus clairement à la lecture de la description qui suit donnant, à titre d'exemple non limitatif et en regard des dessins annexés, un mode de réalisation d'un dispositif d'abaissement local de la température du corps et notamment des jambes respectant les concepts de l'invention.

Cette description se réfère aux dessins annexés sur lesquels :
La figure 1 est une vue de face du dispositif selon l'invention en configuration déployée.
La figure 2 est une vue en perspective du dispositif en configuration fermée et enroulée.

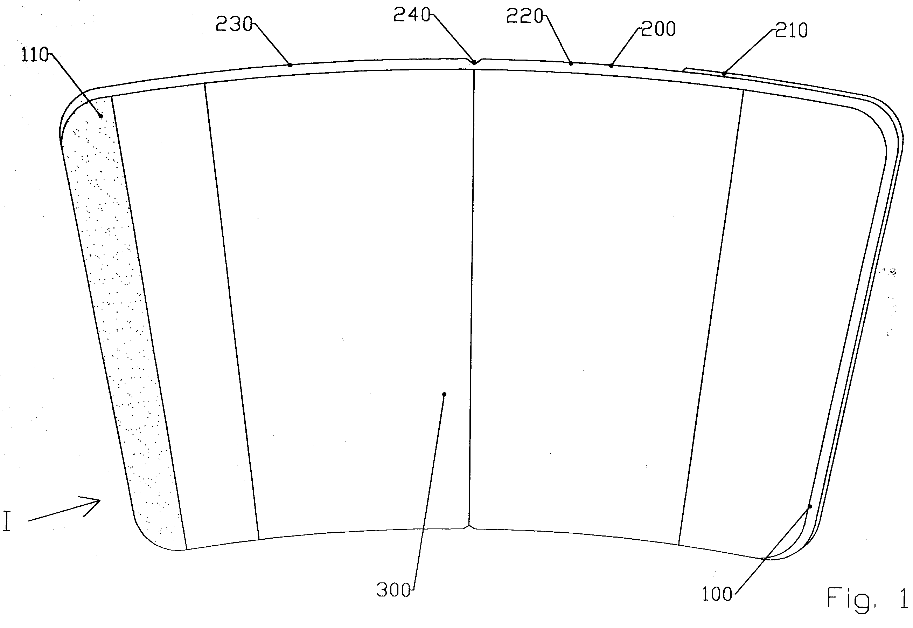

Tel qu'illustré sur le dessin de la figure 1, le dispositif d'abaissement local de la température du corps et notamment de la jambe référencé dans son ensemble I est constitué par deux parois 100 et 200 qui, adoptant la forme d'un secteur angulaire d'une couronne en position déployée, sont liées entre elles de façon à former au moins un compartiment 300 de stockage pour une matière à inertie thermique, lesdites parois 100 et 200 étant liées entre elles de façon à former au moyen de leurs zones de liaison un compartiment 300.

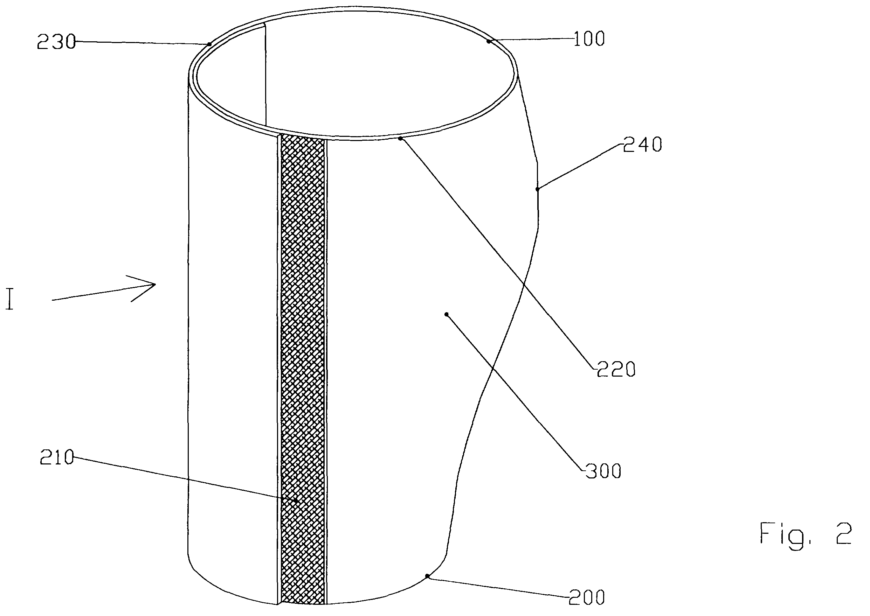

Comme illustré sur le dessin de la figure 2, ledit dispositif I adopte une forme tronconique en position enroulée lui permettant de s'adapter au fuselage naturel des membres du corps. De plus, ledit dispositif I d'abaissement local de la température présente au niveau de sa partie centrale en position enroulée une forme convexe sensiblement identique au profil convexe d'un mollet lui assurant une surface de contact la plus grande possible pour traiter un mollet ou une jambe. L'application du dispositif I sur la zone à traiter permettra de baisser la température dans celle-ci et donc de la soigner. Selon une caractéristique particulièrement avantageuse de l'invention, une poche hermétique thermo-soudée (non représentée) contenant ladite matière à inertie thermique est placée à l'intérieur du dispositif I, dans le compartiment 300 constitué par les deux parois 100 et 200 formant housse et adoptant le profil convexe du dispositif I. En effet privilégiant le confort, les demandeurs ont voulu offrir à l'utilisateur d'un tel dispositif I un contact plus agréable que celui que peut apporter un dispositif en plastique à contact direct sur la peau. De plus, selon une caractéristique particulièrement judicieuse de l'invention, la susdite paroi intérieure 100 de la housse venant en contact avec la jambe est réalisée en tissu élastique anallergique. Ainsi, ce tissu permet non seulement un contact plus agréable mais aussi de s'adapter le mieux possible aux formes avec lesquelles il est en contact et donc de permettre à la poche hermétique et au gel se trouvant à l'intérieur de s'adapter au profil de la zone à traiter.

Selon une caractéristique particulièrement avantageuse de l'invention, la susdite paroi extérieure 200 de la housse est réalisée en tissu élastique et comporte une portion agrippante 210 permettant la fermeture dudit dispositif I autour du mollet, au moyen d'une bande auto-agrippante 110 placée sur la paroi intérieure 100 et sur la longueur d'une extrémité latérale dudit dispositif I et venant se fixer sur ladite portion 210. Selon un mode de réalisation préférée de l'invention, la susdite portion agrippante 210 de la paroi extérieure 200 est constituée par du tissu gratté.

Cette bande large de tissu gratté permet de s'adapter aux différents diamètres de mollets ou de jambes sans avoir à changer de modèle de jambière ou de dispositif I. De plus, un tel dispositif de fixation et maintien en position est plus efficace et esthétique que ceux existant sur le marché. En effet, il n'y a aucune partie en verrue, aucun noeud spécial ou boucle n'est nécessaire pour assurer une bonne fixation tout en permettant de s'adapter à toutes les dimensions de mollets ou jambes.

L'élasticité du tissu constituant la paroi extérieure 200 a pour grand avantage de réaliser en permanence une légère contention de la zone à traiter.

Pour prolonger l'effet du froid sur la zone à soigner, une matière intermédiaire isolante est interposée non représentée, à l'intérieur de la housse, entre ladite paroi extérieure 200 et la poche contenant la matière à inertie thermique. Ainsi, en isolant de l'extérieur la poche de gel, celui-ci restera à une température efficace plus longtemps.

Selon un mode de réalisation préférée de l'invention, la susdite paroi extérieure 200 est constituée de deux parties 220 et 230 de forme symétrique et qui liées entre elles reprennent au niveau de leur ligne de liaison 240 le contour convexe du mollet. Ce découpage particulier des deux parties 220 et 230 de la paroi extérieure 200 permet au dispositif I d'adopter, même lorsque celui-ci n'est pas attaché ou placé sur un mollet, un profil convexe.

Selon un autre mode de réalisation préférée de l'invention mais non limitatif la liaison entre les deux parois 100 et 200 de la housse située au niveau du grand diamètre de la couronne autorise l'ouverture et la fermeture de ladite housse, comme par exemple, une fermeture à glissière ou utilisant un dispositif de type "Velcro". Ce mode de réalisation permet de séparer la poche contenant le gel de la housse en contact direct avec la peau et ainsi permettre le lavage de cette dernière, dans des conditions plus pratiques.

Selon un autre mode de réalisation préférée de l'invention, la liaison entre les deux parois 100 et 200 de la housse située au niveau du grand diamètre de la couronne est réalisée après introduction de la poche hermétique dans la housse à des fins de scellement de l'ouverture.

Le dispositif I pourra être soit directement placé dans le congélateur avant application dans une version où les compartiments seront sans ouverture, soit ceux seront seulement les éléments de matière de conservation du froid qui seront placés dans le congélateur et ensuite placés dans le compartiment prévu à cet effet sur le dispositif I et formant housse avec ouvertures d'accès.

On comprend que le dispositif d'abaissement local de la température du corps qui vient d'être ci-dessus décrit et représenté, l'a été en vue d'une divulgation plutôt que d'une limitation. Bien entendu, divers aménagements, modifications et améliorations pourront être apportés à l'exemple ci-dessus, sans pour autant sortir du cadre de l'invention pris dans ses aspects et dans son esprit les plus larges.

Ainsi par exemple, du tissu éponge pourra être fixé à la paroi intérieure 100 du dispositif I afin d'en améliorer son confort. La paroi extérieure 200 pourra également être constituée dans sa totalité d'un tissu permettant l'adhérence d'une extrémité du dispositif I munie d'une bande d'un autre tissu auto-agrippant.

## Revendications

1. Dispositif (I) d'abaissement local de la température du corps du type de celui, constitué par deux parois (100 et 200) qui, adoptant la forme d'un secteur angulaire d'une couronne en position déployée et une forme tronconique en position enroulée, sont liées entre elles de façon à former au moins un compartiment (300) de stockage pour une matière à inertie thermique, lesdites parois (100 et 200) étant liées entre elles de façon à former au moyen de leurs zones de liaison un ou plusieurs compartiments (300) étanches ou non entre eux, CARACTERISE PAR LE FAIT QU'il présente au niveau de sa partie centrale en position enroulée une forme convexe sensiblement identique au profil convexe d'un mollet.

2. Dispositif (I) selon la revendication 1, CARACTERISE PAR LE FAIT QUE la matière à grande inertie thermique se présente sous la forme d'un gel de polyuréthane.

3. Dispositif (I) selon la revendication 1, CARACTERISE PAR LE FAIT QUE la matière à grande inertie thermique est contenue dans une poche hermétique en thermoplastique extensible du type polyéthylène réalisée par thermo-soudure.

4. Dispositif (I) selon les revendications 1 et 3, CARACTERISE PAR LE FAIT QUE la poche hermétique thermo-soudée contenant ladite matière à inertie thermique est placée à l'intérieur du dispositif, dans le compartiment (300) constitué par les deux parois (100 et 200) formant housse et adoptant le profil convexe du dispositif.

5. Dispositif (I) selon les revendications 1, 3 et 4, CARACTERISE PAR LE FAIT QUE la susdite paroi intérieure (100) de la housse venant en contact avec la jambe est réalisée en tissu élastique anallergique.

6. Dispositif (1) selon les revendications 1, 3, 4 et 5 prises ensemble, CARACTERISE PAR LE FAIT QUE la susdite paroi extérieure (200) de la housse est réalisée dans un tissu élastique et comporte une portion agrippante (210) permettant la fermeture dudit dispositif (I) autour du mollet, au moyen d'une bande auto-agrippante (110) placée sur la paroi intérieure (100) et sur la longueur d'une extrémité latérale dudit dispositif (I) et venant se fixer sur ladite portion (210).

7. Dispositif (I) selon les revendications 1, 3, 4, 5 et 6 prises ensemble, CARACTERISE PAR LE FAIT QU' une matière intermédiaire isolante est interposée, à l'intérieur de la housse, entre ladite paroi extérieure (200) et la poche contenant la matière à inertie thermique.

8. Dispositif (I) selon les revendications 1 et 6, CARACTERISE PAR LE FAIT QUE la susdite paroi extérieure (200) est constituée de deux parties (220 et 230) de forme symétrique et qui liées entre elles reprennent au niveau de leur ligne de liaison le contour convexe du mollet.

9. Dispositif (I) selon l'une quelconque des revendications 1 à 8, CARACTERISE PAR LE FAIT QUE la liaison entre les deux parois (100 et 200) de la housse située au niveau du grand diamètre de la couronne autorise l'ouverture et la fermeture de ladite housse.

10. Dispositif (I) selon l'une quelconque des revendications 1 à 9, CARACTERISE PAR LE FAIT QUE la liaison entre les deux parois (100 et 200) de la housse située au niveau du grand diamètre de la couronne est réalisée après introduction de la poche hermétique dans la housse à des fins de scellement de l'ouverture.
